# EUROPEAN PATENT SPECIFICATION

(11) **EP 2 874 611 B1**
(45) Date of publication and mention of the grant of the patent: **14.09.2016**
(21) Application number: 13739677.6
(22) Date of filing: 18.07.2013
(51) Int. Cl.: A61K 9/20, A61K 31/192, A61K 31/196, A61K 31/4439

(54) **GASTRO-RESISTANT DELAYED RELEASE PHARMACEUTICAL FORM**
MAGENRESISTENTE PHARMAZEUTISCHE FORM MIT VERZÖGERTER FREISETZUNG
FORMULATION PHARMACEUTIQUE GASTRO-RÉSISTANTE À LIBÉRATION RETARDÉE

(30) Priority: 19.07.2012 IT MI20121264
(43) Date of publication of application: 27.05.2015
(73) Proprietor: Recordati Industria Chimica E Farmaceutica SPA, 20148 Milano (IT)
(72) Inventor: COLOMBO, Paolo, I-43121 Parma (IT); ROSSI, Alessandra, I-43121 Parma (IT); BARBIERI, Stefano, I-26100 Cremona (IT); CONTI, Chiara, I-29010 Vigolo Marchese (IT)
(74) Representative: Longoni, Alessandra
(86) International application number: PCT/EP2013/065169
(87) International publication number: WO 2014/013008

(56) References cited:
- WO-A1-93/13138
- WO-A1-2009/130394

## Description

The present invention relates to a gastro-resistant delayed release pharmaceutical form and, more particularly, to a solid gastro-resistant pharmaceutical form obtained without using any gastro-resistant film coating.

The need to prepare gastro-resistant dosage forms often occurs in pharmaceutical technology for absorption as well as for tolerability reasons. For example, gastro-resistant tablets are needed when the active ingredient to administer by oral route has a poor solubility in acid environment and must then avoid the contact with the gastric environment not to degrade. A typical example is represented by proton pump inhibitors (PPI).

Analogously it is necessary to avoid the contact with the gastric environment when the active ingredient may be detrimental to the stomach. In this case gastro-resistance may be considered as a form of gastro-protection. A typical example is represented by non-steroid anti-inflammatory agents (NSAID).

In case of gastro-resistance as well as in case of gastro-protection, the dosage form is generally prepared with methods based on film coating techniques of a solid core such as granules, tablets or capsules. These film coating techniques provides that the solid cores containing the active ingredient are coated with an impermeable polymeric film which can resist in acid solutions without dissolving. The coating process implies several steps of spraying of the polymeric solution on the cores and subsequent drying. These sequential steps of spraying and drying are performed until the thickness of the film reaches a value which can protect the core from the acid solution and prevent the release of the active ingredient at acid pH. When the pH changes and reaches the value of the first tract of the intestine (around pH 5.0), these gastro-resistant films dissolve and allow the release of the active ingredient from the pharmaceutical form.

Representative prior art are WO 93/13138 A1 and WO 2009/130394 A1.

The gastro-resistant film coating, even if widely used, shows several drawbacks.

The industrial preparation of these dosage forms is particularly long and can be used only for solid dosage forms such tablets, pellets and granules. In case of dosage forms prepared for the combination therapy with more than one active ingredient, the film coating of the tablet can be used only when all the active ingredients must avoid the contact with the gastric environment. In case of combinations of active ingredients requiring different release kinetics, multilayered tablets wherein the different active ingredients are put in separated layers of the multilayered core are often used; also in this case it is very difficult to obtain a coating of a single layer containing the active ingredient which need gastro-resistance or gastro-protection without coating also the other layers. For example, in a three layered tablet wherein one layer contains a drug for gastro-retentive release, the central layer contains a drug for immediate release and the third layer contains a drug for delayed release, the achievement of the delayed release from this layer without affecting the other two layers is a problem which cannot be solved by a film coating technique.

We have found the possibility to prepare gastro-resistant dosage forms by compression of a powder mixture without requiring film coating.

Therefore, object of the present invention is a solid gastro-resistant pharmaceutical form containing a gastro-resistant matrix consisting of a mixture of three hydrophilic polymers wherein the first polymer is swellable and pH-independent, the second polymer is swellable, insoluble at pH lower than 4.0 and soluble at pH higher than 4.0, and the third polymer is an oligomer able to make a soluble complex with the active ingredient.

The combined use of the three hydrophilic polymers according to the invention allows to very efficiently delay the release of the active ingredient from the gastric tract, so obtaining a gastro-resistant pharmaceutical form.

Therefore, a further object of the present invention is the use of a mixture of three hydrophilic polymers wherein the first polymer is swellable and pH-independent, the second polymer is swellable, insoluble at pH lower than 4.0 and soluble at pH higher than 4.0, and the third polymer is an oligomer able to make a soluble complex with the active ingredient for the preparation of a gastro-resistant matrix of pharmaceutical forms devoid of gastro-resistant film coating.

Said three polymers have a synergic effect because they form together a strong transient barrier of gelled polymer. The gelled barrier reduces the diffusion of the active ingredient when the dosage form containing these polymers is immersed in an acid solution. On the contrary, the gelled barrier dissolves at an intestinal pH from 4.5 to 7.4.

The mixture of three hydrophilic polymers according to the invention is used in the form of powder and then can be mixed with other conventional excipients and compressed to obtain the desired gastro-resistant pharmaceutical form. The gastro-resistant film coating is not needed making the manufacturing process easier.

Moreover, also in case of multilayered tablets, it is possible to have a single gastro-resistant layer by adding the mixture of the three hydrophilic polymers according to the invention to the composition of said layer.

Without being bound to any theory, the Inventors believe that the combined use of the three hydrophilic polymers according to the invention results in the observed gastro-resistant effect in the pharmaceutical forms containing them according to the following mechanism.

When the tablet or the layer of the multilayered tablet, containing the mixture of the three hydrophilic polymers according to the invention, enters in contact with an acid solution, the hydrophilic pH-independent polymer rapidly jellifies, increases in volume and creates a prompt and resistant gelled barrier that coats the core not yet reached by the solvent. This gelled layer strongly delays the entrance of the liquid into the core acting as a physical barrier to the diffusion of water. At the same time, the barrier of gelled polymer is an obstacle for the diffusion of the active ingredient within the core to the outside, so protecting the gastric mucosa from the contact with the active ingredient in case gastro-protection is desired or protecting the active ingredient from the gastric acid environment in case gastro-resistance is desired.

The diffusion of the active ingredient to the outside is also prevented by the presence of the hydrophilic oligomer, that complexes the eventually dissolved molecules of the active ingredient, in the core and in the gel. During this process of wetting, swelling and liquid transport, the polymer that is insoluble in the acid medium remains unchanged after contact with the liquid. This means that this polymer insoluble in acid environment does not swell or dissolve. Practically, it does not contribute to the gelled layer since the pH conditions do not allow its structure to interact with the water in the stomach. Thus, in the acidic conditions the pH-dependent polymer helps to maintain the core compact and dry. As a consequence, the release of the active ingredient, in particular when it has a low solubility in acid, is practically absent. When the dosage form is transported in the intestine where the pH increases from 4.5 to 7.4, also the pH-dependent polymer swells and jellifies.

The swelling and dissolution of this polymer is much faster than the first one. As a consequence, the dosage form rapidly disintegrates by dissolution or erosion making available for absorption the active ingredient present in the core in undissolved form or as a complex with the oligomer polymer. Then, only at intestinal level, the active ingredient contained in the dosage form can dissolve and release out of the completely swelled or disintegrated solid core.

Among the hydrophilic polymers which can be used in the mixture according to the present invention particularly suitable are:
swellable pH-independent hydrophilic polymers such as cellulose derivatives, for example hydroxypropylmethylcellulose, hydroxypropylcellulose, hydroxyethylcellulose, scleroglucans or non-ionic polymers such as polyethylenoxide;
swellable pH-dependent hydrophilic polymers such as methacrylic polymers soluble at basic pH, cellulose acetophthalate, hydroxypropylmethylcellulose phthalate, sodium alginate;
hydrophilic oligomer polymers such as cyclodextrin derivatives like α-cyclodextrin, β-cyclodextrin, γ-cyclodextrin, methyl-β-cyclodextrin and hydroxypropyl-β-cyclodextrin. Hydroxypropylmethylcellulose, particularly high molecular weight hydroxypropylmethylcellulose, which show better gelling properties, is preferred among the swellable pH-independent hydrophilic polymers.

Specific examples of high molecular weight hydroxypropylmethylcellulose (HPMC) are HPMC having molecular weight from 10000 to 1000000 Da sold under the trademark Methocel.

Sodium alginate is particularly preferred among the swellable pH-dependent hydrophilic polymers, in particular its forms more rich in mannuronic acid (M-rich) which allow higher drug release rates at pH 6.8 and hydrate faster under acid condition in comparison with the forms more rich in guluronic acid (G-rich).

B-cyclodextrin is preferred among the hydrophilic oligomer polymers.

The weight ratios among the three polymers (swellable pH-independent hydrophilic polymers, swellable pH-dependent hydrophilic polymers and hydrophilic oligomer polymers) are generally from 1:1:1 to 3:1:2, preferably from 1:1:1 to 3:1:1; still more preferably the three polymers are used in equal weight ratios (1:1:1).

However, depending on the physico-chemical properties of the active ingredient, the weight ratio among the three hydrophilic polymers can be optimized outside said ranges but, in this case, the sum of the three numbers of the ratio is equal to 3.

The weight of each hydrophilic polymer in the dosage form is generally from 5% to 70% w/w, preferably from 10% to 60% w/w, still more preferably from 10% to 40% w/w.

By mixing these three polymers, in the appropriate amounts and ratios as above explained, with the active ingredient or the mixture of active ingredients and other conventional excipients for the preparation of solid forms a powder mixture is obtained which, compressed into tablets or multilayered tablets, gives a gastro-resistant solid dosage form.

The preparation of the gastro-resistant pharmaceutical forms according to the present invention is then carried out according to conventional techniques in pharmaceutical technology such as mixing, compression, etc.

The mixture of polymers according to the present invention can be advantageously used for the formulation of any active ingredient which needs gastro-resistance or gastro-protection.

Specific examples of such active ingredients are proton pump inhibitors, such as omeoprazole, esomeprazole, pantoprazole and lansoprazole, non steroidal anti-inflammatory agents, such as flurbiprofen, piroxicam, meloxicam, diclofenac, naproxen and ketoprofen, steroidal anti-inflammatory agents such as prednisolone.

### Brief description of the figures

Figure 1- dissolution profile of a cylindrical tablet containing flurbiprofen, prepared as described in example 1
Figure 2 - dissolution profile of a cylindrical tablet containing flurbiprofen, prepared as described in example 2

In order to better illustrate the present invention without limiting it, the following examples are now given.

### Example 1

### Cylindrical tablet containing flurbiprofen 37.5 mg.

To a granulate of flurbiprofen, mannitol and polyvinylpyrrolidone, prepared by wet granulation, mannitol, sodium alginate, hydroxypropylmethylcellulose and β-cyclodextrin were added. The powder was mixed in Turbula for 15 minutes. Subsequently magnesium stearate and yellow lake were added by mixing for further 5 minutes. The resultant mixture was compressed using cylindrical 10 mm punches to obtain a tablet weighing 307 mg.

| Composition | | |
|---|---|---|
| Ingredients | mg | % w/w |
| Flurbiprofen | 37.5 | 12.21 |
| Mannitol | 26.10 | 8.50 |
| Polyvinylpyrrolidone (PVP K30) | 2.64 | 0.86 |
| Mannitol | 34.58 | 11.27 |
| β-cyclodextrin | 68.09 | 22.18 |
| Sodium alginate | 68.09 | 22.18 |
| Hydroxypropylmethylcellulose (Methocel K4M) | 68.09 | 22.18 |
| Magnesium stearate | 1.51 | 0.13 |
| Yellow lake | 0.40 | 0.13 |

### Example 2

### Cylindrical tablet containing flurbiprofen 37.5 mg.

By working in a way similar to that described in example 1, cylindrical tablets containing flurbiprofen were prepared. With respect to example 1, in this preparation the composition was changed to obtain a slower delayed flurbiprofen release.

| Composition | | |
|---|---|---|
| Ingredients | mg | % w/w |
| Flurbiprofen | 37.5 | 12.21 |
| Mannitol | 26.10 | 8.50 |
| Polyvinylpyrrolidone (PVP K30) | 2.64 | 0.86 |
| Mannitol | 34.58 | 11.27 |
| β-cyclodextrin | 68.09 | 22.18 |
| Sodium alginate | 34.05 | 11.09 |
| Hydroxypropylmethylcellulose (Methocel K4M) | 102.13 | 33.27 |
| Magnesium stearate | 1.51 | 0.49 |
| Yellow lake | 0.40 | 0.13 |

### Example 3

### Dissolution test

The dissolution profile of the cylindrical tablets prepared as described in examples 1 and 2 was determined by immersion in simulated gastric fluid.

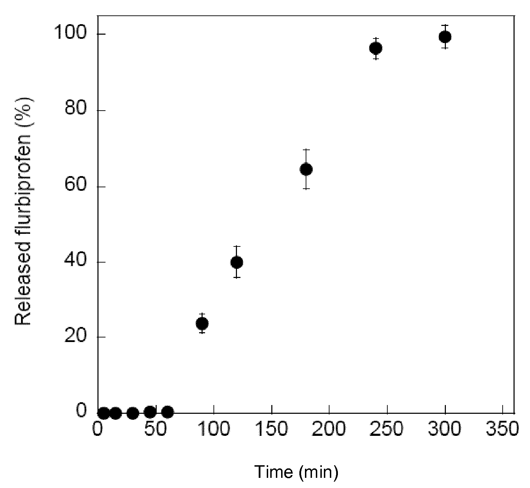

After immersion into the vessel (Apparatus 2 USPXXXIV ed., 100 rpm), the tablets placed themselves on the bottom of the vessel. Flurbiprofen was not released during the first hour at pH 1.2. After having raised the pH to 7.2 the release developed in a linear way reaching 100% in 5 hours. In Figure 1 the dissolution profile of flurbiprofen of the tablets of example 1 is graphically reported.

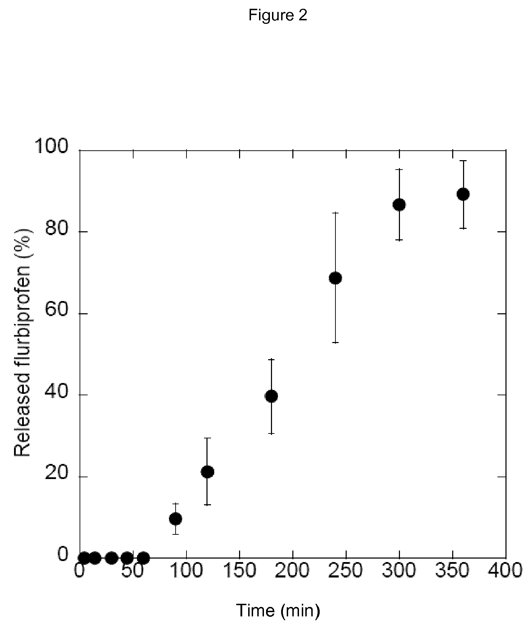

In Figure 2 the dissolution profile of flurbiprofen of the tablets of example 2 is graphically reported. Also in this case flurbiprofen was not released at pH 1.2. After having raised the pH to 7.2 the release developed in a linear way reaching 90% in 6 hours.

### Example 4

### Cylindrical tablet containing diclofenac 75 mg.

To a granulate of diclofenac, mannitol and polyvinylpyrrolidone, prepared by wet granulation, sodium alginate, hydroxypropylmethylcellulose and β-cyclodextrin were added. The powder was mixed in Turbula for 15 minutes. Subsequently magnesium stearate and yellow lake were added by mixing for further 5 minutes. The resultant mixture was compressed using cylindrical 10 mm punches to obtain a tablet weighing 447.67 mg.

| Composition | | |
|---|---|---|
| Ingredients | mg | % w/w |
| Diclofenac potassium | 84.90 | 18.97 |
| Mannitol | 59.08 | 13.20 |
| Polyvinylpyrrolidone (PVP K30) | 3.99 | 0.89 |
| β-cyclodextrin | 99.00 | 22.11 |
| Sodium alginate | 99.00 | 22.11 |
| Hydroxypropylmethylcellulose (Methocel K4M) | 99.00 | 22.11 |
| Magnesium stearate | 2.20 | 0.49 |
| Yellow lake | 0.55 | 0.12 |

### Example 5

### Cylindrical tablet containing diclofenac 75 mg.

By working in a way similar to that described in example 4, cylindrical tablets containing diclofenac were prepared. With respect to example 4, in this preparation the composition was changed to obtain a slower delayed diclofenac release.

| Composition | | |
|---|---|---|
| Ingredients | mg | % w/w |
| Diclofenac potassium | 84.90 | 18.97 |
| Mannitol | 59.08 | 13.20 |
| Polyvinylpyrrolidone (PVP K30) | 3.99 | 0.89 |
| β-cyclodextrin | 99.00 | 22.11 |
| Sodium alginate | 49.50 | 11.06 |
| Hydroxypropylmethylcellulose (Methocel K4M) | 148.50 | 33.16 |
| Magnesium stearate | 2.20 | 0.49 |
| Yellow lake | 0.55 | 0.12 |

### Example 6

### Dissolution test

The dissolution profile of the cylindrical tablets prepared as described in examples 4 and 5 was determined by immersion in simulated gastric fluid.

After immersion into the vessel (Apparatus 2 USPXXXIV ed., 100 rpm), the tablets placed themselves on the bottom of the vessel. Diclofenac was not released during the first hour at pH 1.2. After having raised the pH to 7.2 the release developed in a linear way reaching 100% in 5 hours for the tablets of example 4 and 90% in 6 hours for the tablets of example 5.

### Example 7

### Cylindrical tablet containing esomeprazole 20 mg.

To a granulate of esomeprazole, prepared by dry granulation, mannitol, sodium alginate, hydroxypropylmethylcellulose and β-cyclodextrin were added. The powder was mixed in Turbula for 15 minutes. Subsequently magnesium stearate and yellow lake were added by mixing for further 5 minutes. The resultant mixture was compressed using cylindrical 10 mm punches to obtain a tablet weighing 262.76 mg.

| Composition | | |
|---|---|---|
| Ingredients | mg | % w/w |
| Esomeprazole magnesium dihydrate | 22.00 | 8.30 |
| Mannitol | 34.58 | 13.50 |
| β-cyclodextrin | 68.09 | 25.90 |
| Sodium alginate | 68.09 | 25.90 |
| Hydroxypropylmethylcellulose (Methocel K4M) | 68.09 | 25.90 |
| Magnesium stearate | 1.51 | 0.40 |
| Yellow lake | 0.40 | 0.10 |

### Example 8

### Dissolution test

The dissolution profile of the cylindrical tablets prepared as described in example 7 was determined by immersion in simulated gastric fluid.

After immersion into the vessel (Apparatus 4 USPXXXIV ed.), esomeprazole was not released during the first hour at pH 1.2. After having raised the pH to 7.2 the release developed in a linear way reaching 90% in 5 hours.

## Claims

1. A solid gastro-resistant pharmaceutical form containing a gastro-resistant matrix which consists of a mixture of three hydrophilic polymers wherein the first polymer is a swelling pH-independent polymer, the second polymer is a swelling polymer insoluble at a pH lower than 4.0 and soluble at a pH higher than 4.0, and the third polymer is an oligomer able to make a soluble complex with the active ingredient.

2. A pharmaceutical form according to claim 1 wherein the swelling pH-independent polymer is selected among cellulose derivatives, scleroglucan and polyethylenoxide.

3. A pharmaceutical form according to claim 2 wherein the swelling pH-independent polymer is a cellulose derivative selected among hydroxypropylmethylcellulose, hydroxypropylcellulose and hydroxyethylcellulose.

4. A pharmaceutical form according to claim 3 wherein the swelling pH-independent polymer is hydroxypropylmethylcellulose.

5. A pharmaceutical form according to claim 1 wherein the second swelling polymer is selected among methacrylic polymers soluble at a basic pH, cellulose acetophthalate, hydroxypropylmethylcellulose phthalate and sodium alginate.

6. A pharmaceutical form according to claim 1 wherein the second swelling polymer is sodium alginate.

7. A pharmaceutical form according to claim 1 wherein the oligomer is a cyclodextrin derivative selected among α-cyclodextrin, β-cyclodextrin, γ-cyclodextrin, methyl-β-cyclodextrin and hydroxypropyl-β-cyclodextrin.

8. A pharmaceutical form according to claim 7 wherein the oligomer is β-cyclodextrin.

9. A pharmaceutical form according to claim 1 wherein the weight ratio first polymer:second polymer:third polymer is from 1:1:1 to 3:2:1.

10. Use of a mixture of three hydropilic polymers wherein the first polymer is a swelling pH-independent polymer, the second polymer is a swelling polymer insoluble at a pH lower than 4.0 and soluble at a pH higher than 4.0, and the third polymer is an oligomer able to make a soluble complex with the active ingredient for the preparation of a gastro-resistant matrix of pharmaceutical forms without gastro-resistant film coating.

## Patentansprüche

1. Eine solide, magensaftresistente pharmazeutische Form, enthaltend eine magensaftresistente Matrix, die aus einem Gemisch von drei hydrophilen Polymeren besteht, wobei das erste Polymer ein anschwellendes, pH-unabhängiges Polymer ist, das zweite Polymer ein anschwellendes Polymer ist, welches bei einem niedrigeren pH-Wert als 4,0 unlöslich ist und bei einem höheren pH-Wert als 4,0 löslich ist und das dritte Polymer ein Oligomer ist, das dazu geeignet ist, mit dem Wirkstoff einen löslichen Komplex zu bilden.

2. Eine pharmazeutische Form gemäß Anspruch 1, wobei das anschwellende, pH-unabhängige Polymer unter Zellulose-Derivaten, Scleroglucan und Polyethylenoxid ausgewählt ist.

3. Eine pharmazeutische Form gemäß Anspruch 2, wobei das anschwellende, pH-unabhängige Polymer ein Zellulose-Derivat unter folgenden ist: Hydroxypropylmethylcellulose, Hydroxypropylcellulose und Hydroxyethylcellulose.

4. Eine pharmazeutische Form gemäß Anspruch 3, wobei das anschwellende, pH-unabhängige Polymer Hydroxypropylmethylcellulose ist.

5. Eine pharmazeutische Form gemäß Anspruch 1, wobei das zweite anschwellende Polymer unter Methacrylatpolymeren, die bei einem basischen pH-Wert löslich sind, sowie Cellulose-Acetat-Phthalat, Hydroxypropylmethylcellulose-Phthalat und Natriumalginat ausgewählt wird.

6. Eine pharmazeutische Form gemäß Anspruch 1, wobei das zweite anschwellende Polymer Natriumalginat ist.

7. Eine pharmazeutische Form gemäß Anspruch 1, wobei das Oligomer ein Cyclodextrin-Derivat unter folgenden ist: α-Cyclodextrin, β-Cyclodextrin, γ-Cyclodextrin, Methyl-β-Cyclodextrin und Hydroxypropyl-β-Cyclodextrin.

8. Eine pharmazeutische Form gemäß Anspruch 7, wobei das Oligomer β-Cyclodextrin ist.

9. Eine pharmazeutische Form gemäß Anspruch 1, wobei das Gewichtsverhältnis "erstes Polymer:zweites Polymer:drittes Polymer" von 1:1:1 bis 3:2:1 geht.

10. Verwendung eines Gemischs aus drei hydrophilen Polymeren, wobei das erste Polymer ein anschwellendes, pH-unabhängiges Polymer ist, das zweite Polymer ein anschwellendes Polymer ist, welches bei einem niedrigeren pH-Wert als 4,0 unlöslich und bei einem höheren pH-Wert als 4,0 löslich ist und das dritte Polymer ein Oligomer ist, dazu geeignet, mit dem Wirkstoff zur Zubereitung einer magensaftresistenten Matrix aus pharmazeutischen Formen ohne magensaftresistenten Filmüberzug einen löslichen Komplex zu bilden.

## Revendications

1. Forme pharmaceutique solide gastro-résistante contenant une matière composite gastro-résistante qui consiste en un mélange de trois polymères hydrophiles dans lesquels le premier polymère est un polymère gonflant indépendant du pH, le deuxième polymère est un polymère gonflant insoluble à un pH inférieur à 4,0 et soluble à un pH supérieur à 4,0, et le troisième polymère est un oligomère susceptible de donner un complexe soluble avec l'ingrédient actif.

2. Forme pharmaceutique selon la revendication 1, dans laquelle le polymère gonflant indépendant du pH est sélectionné parmi des dérivés de cellulose, le scléroglucane et l'oxyde de polyéthylène.

3. Forme pharmaceutique selon la revendication 2, dans laquelle le polymère gonflant indépendant du pH est un dérivé de cellulose sélectionné parmi l'hydroxypropylméthylcellulose, l'hydroxypropylcellulose et l'hydroxyéthylcellulose.

4. Forme pharmaceutique selon la revendication 3, dans laquelle le polymère gonflant indépendant du pH est de l'hydroxypropylméthylcellulose.

5. Forme pharmaceutique selon la revendication 1, dans laquelle le deuxième polymère gonflant est sélectionné parmi des polymères méthacryliques solubles à un pH basique, l'acétophtalate de cellulose, le phtalate d'hydroxypropylméthylcellulose et l'alginate de sodium.

6. Forme pharmaceutique selon la revendication 1, dans laquelle le deuxième polymère gonflant est de l'alginate de sodium.

7. Forme pharmaceutique selon la revendication 1, dans laquelle l'oligomère est un dérivé de cyclodextrine sélectionné parmi une α-cyclodextrine, β-cyclodextrine, γ-cyclodextrine, méthyl-β-cyclodextrine et hydroxypropyl-β-cyclodextrine.

8. Forme pharmaceutique selon la revendication 7, dans laquelle l'oligomère est de la β-cyclodextrine.

9. Forme pharmaceutique selon la revendication 1, dans laquelle le rapport pondéral premier polymère:deuxième polymère:troisième polymère va de 1:1:1 à 3:2:1.

10. Utilisation d'un mélange de trois polymères hydrophiles dans lesquels le premier polymère est un polymère gonflant indépendant du pH, le deuxième polymère est un polymère gonflant insoluble à un pH inférieur à 4,0 et soluble à un pH supérieur à 4,0, et le troisième polymère est un oligomère susceptible de donner un complexe soluble avec l'ingrédient actif pour la préparation d'une matière composite gastro-résistante de formes pharmaceutiques sans revêtement de film gastro-résistant.
